# EUROPEAN PATENT SPECIFICATION

(11) **EP 3 947 331 B1**
(45) Date of publication and mention of the grant of the patent: **31.05.2023**
(21) Application number: 20722782.8
(22) Date of filing: 03.04.2020
(51) Int. Cl.: C07C 17/25, C07C 17/269, C07C 17/354, C07C 21/20, C07C 21/22

(54) **PROCESSES FOR PRODUCING Z-1,1,1,4,4,4-HEXAFLUOROBUT-2-ENE AND INTERMEDIATES FOR PRODUCING SAME**
VERFAHREN ZUR HERSTELLUNG VON Z-1,1,1,4,4,4-HEXAFLUORBUT-2-EN UND ZWISCHENPRODUKTE ZUR HERSTELLUNG DAVON
PROCÉDÉS DE PRODUCTION DE Z-1,1,1,4,4,4-HEXAFLUOROBUT-2-ÈNE ET INTERMÉDIAIRES POUR LEUR PRODUCTION

(30) Priority: 05.04.2019 US 201962829856 P
(43) Date of publication of application: 09.02.2022
(73) Proprietor: The Chemours Company FC, LLC, Wilmington, Delaware 19801 (US)
(72) Inventor: PENG, Sheng, Hockessin, Delaware 19707 (US); SIEVERT, Allen Capron, Elkton, Maryland 21921 (US)
(74) Representative: Morf, Jan Stefan
(86) International application number: PCT/US2020/026612
(87) International publication number: WO 2020/206279

(56) References cited:
- WO-A1-2019/023572
- CN-A- 105 218 296

## Description

### TECHNICAL FIELD

The disclosure herein relates to processes for producing Z-1,1,1,4,4,4-hexafluoro-2-butene, and intermediates useful its production. The disclosure further provides processes for producing E- and/or Z-1,1,1,4,4,4-hexafluoro-2-chloro-2-butene.

### BACKGROUND

Many industries have been working for the past few decades to find replacements for the ozone depleting chlorofluorocarbons (CFCs) and hydrochlorofluorocarbons (HCFCs). The CFCs and HCFCs have been employed in a wide range of applications, including their use as refrigerants, cleaning agents, expansion agents for thermoplastic and thermoset foams, heat transfer media, gaseous dielectrics, aerosol propellants, fire extinguishing and suppression agents, power cycle working fluids, polymerization media, particulate removal fluids, carrier fluids, buffing abrasive agents, and displacement drying agents. In the search for replacements for these versatile compounds, many industries have turned to the use of hydrofluorocarbons (HFCs). HFCs have zero ozone depletion potential and thus are not affected by the current regulatory phase-out as a result of the Montreal Protocol.

In addition to ozone depleting concerns, global warming is another environmental concern in many of these applications. Thus, there is a need for compositions that meet both low ozone depletion standards as well as having low global warming potentials. Certain hydrofluoroolefins are believed to meet both goals. Thus there is a need for manufacturing processes that provide intermediates useful to produce hydrofluoroolefins and hydrofluoroolefins that contain no chlorine, which hydrofluoroolefins have low global warming potential. WO 2019/023572 discloses a process for the production of 1,1,1 ,4,4,4-hexafluoro-2-chloro-2-butene from hexachlorobutadiene.

### SUMMARY

The present disclosure provides processes for the production of hydrofluoroolefin Z-1,1,1,4,4,4-hexafluorobut-2-ene (Z-HFO-1336mzz, or Z-1336mzz) and intermediates useful its production.

The present disclosure further provides a process for the production of a product mixture comprising E- and/or Z-1,1,1,4,4,4-hexafluoro-2-chloro-2-butene (HCFO-1326mxz, 1326mxz) comprising contacting 1,1,2,4,4-pentachlorobuta-1,3-diene (HCC-2320az) with HF and a fluorination catalyst.

The present disclosure provides a process for the production of Z-1,1,1,4,4,4-hexafluorobut-2-ene comprising (a) contacting 1,1,2,4,4-pentachlorobuta-1,3-diene with HF in the vapor phase in the presence of a chlorine source and a fluorination catalyst to produce a product mixture comprising *E*- and/or *Z*-1,1,1,4,4,4-hexafluoro-2-chloro-2-butene; (b) contacting *E*- and/or *Z*-1,1,1,4,4,4-hexafluoro-2-chloro-2-butene with base to produce a product mixture comprising 1,1,1,4,4,4-hexafluoro-2-butyne; and (c) contacting 1,1,1,4,4,4-hexafluoro-2-butyne with hydrogen to produce a product mixture comprising *Z*-1,1,1,4,4,4-hexafluoro-2-butene.

In some embodiments, 1,1,2,4,4-pentachlorobuta-1,3-diene (HCC-2320az, 2320az) is produced according to a process comprising dimerization of trichloroethylene (TCE). A process to produce 2320az comprises contacting TCE in the presence of a catalyst to produce a product mixture comprising 2320az.

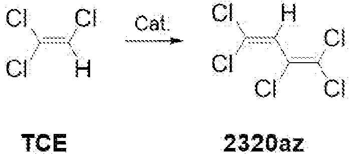

In some embodiments, the dimerization of TCE is performed in the presence of pentachloroethane (CCl₃CHCl₂, HCC-120), which accelerates the dimerization process.

In certain embodiments, 2320az is produced with a selectivity of at least 80%; in some embodiments, selectivity is greater than 90% or greater than 95% or greater than 99% or greater than 99.5%. In certain embodiments, 2320az is recovered from the product mixture. In some embodiments, unreacted TCE is recovered and recycled.

The present disclosure further provides compositions produced according to the processes disclosed herein.

### DETAILED DESCRIPTION

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus.

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

By "recovering" it is meant to sufficiently isolate the desired product to make it available for its intended use, either as a starting material for a subsequent reaction step or, in the case of recovering Z-1,1,1,4,4,4-hexafluoro-2-butene, useful, for example, as a refrigerant or foam expansion agent.

The details of the recovery step will depend on the compatibility of the product mixture with the reaction conditions of the subsequent reaction step. For example, if the product is produced in a reaction medium that is different from or incompatible with a subsequent reaction step, then the recovery step may include separation of the desired product from the product mixture including the reaction medium. This separation may occur simultaneously with the contacting step when the desired product is volatile under the reaction conditions. The volatilization of the desired product can constitute the isolation and thereby the recovery of the desired product. If the vapors include other materials intended for separation from the desired product, the desired product may be separated, by selective distillation, for example.

The steps for recovering the desired product from the product mixture, preferably comprise separating the desired product from catalyst or other component(s) of the product mixture used to produce the desired product or produced in the process.

The present disclosure provides, inter alia, processes to produce Z-1336mzz, and intermediates for producing Z-1336mzz. Such process may use a starting material comprising 1,1,2,4,4-pentachlorobuta-1,3-diene, which may be produced from trichloroethylene, one method as set forth herein.

### Production of 1,1,2,4,4-pentachlorobuta-1,3-diene (2320az)

1,1,2,4,4-pentachlorobuta-1,3-diene (HCC-2320az, or 2320az) may be produced in accordance with this disclosure by dimerization of trichloroethylene (TCE). In some embodiments, there is provided a process to produce a product mixture comprising 2320az, which process comprises contacting TCE with a dimerization catalyst at an elevated temperature.

In some embodiments, the dimerization catalyst comprises iron. An iron dimerization catalyst may comprise metallic iron from any source (including a combination of sources) and may be or comprise iron powder, iron wire, iron screen or iron turnings. The iron catalyst may also comprise an iron salt such as ferric chloride or ferrous chloride (FeCl₃ or FeCl₂, respectively).

In some embodiments, the dimerization catalyst comprises copper. A copper dimerization catalyst may comprise metallic copper from any source (including a combination of sources) and may be or comprise copper powder or copper wire, for example. The copper catalyst may also comprise a cuprous or a cupric salt such as cuprous chloride or cupric chloride (CuCl or CuCl₂, respectively).

The process is preferably performed in an anhydrous environment. For example, when ferric chloride is used, the ferric chloride is preferably anhydrous.

In some embodiments, the dimerization catalyst has a particular concentration with respect to moles of TCE reactant used. As such, in some embodiments wherein the catalyst comprises a metallic iron catalyst, a ratio of weight of Fe wire (or Fe powder) catalyst to TCE is from about 0.0001 to about 1. In other embodiments, the weight ratio of iron catalyst to TCE is from about 0.01 to about 1.

In some embodiments, the dimerization catalyst comprises ferric chloride and the weight ratio of ferric chloride to TCE is from about 0.00001 to about 1. For example, the weight ratio of ferric chloride to TCE is from about 0.00001 to about 0.002, while in another example, the weight ratio is from about 0.00005 to about 0.001. In yet another example, a weight ratio of ferric chloride to TCE is from about 0.0001 to about 1, while in a further example, the ratio of ferric chloride to TCE is from about 0.00015 to about 1.

In some embodiments, trichloroethylene is contacted with a dimerization catalyst and pentachloroethane. Pentachloroethane (HCC-120) accelerates the reaction to produce the product mixture comprising 2320az. In certain embodiments, a weight ratio of HCC-120 to TCE is from about 0.001 to about 1. In other embodiments, the weight ratio of HCC-120 to TCE is from about 0.005 to about 1.

The dimerization of TCE is performed in at an elevated temperature, for example at a temperature in the range of about 210 to about 235°C. The temperature may be greater than 200°C. The temperature may be less than 245°C.

Pressure is typically autogenous.

Contact (residence) time is typically about 0.5 to 10 hours.

In some embodiments, conversion of TCE is at least 15% or at least 30%, or at least 50%. In some embodiments, selectivity to 2320az is at least 80%, or at least 85%, or at least 90%.

Byproducts in the dimerization reaction may include tetrachloroethane isomers, tetrachlorobutadiene isomers, hexachlorobutene isomers, trichloroethylene oligomers. The product mixture comprising 2320az may further comprise E-1,1,2,3,4-pentachloro-1,3-butadiene or Z-1,1,2,3,4-pentachloro-1,3-butadiene. Thus, in one embodiment there is a composition comprising 1,1,2,4,4-pentachlorobuta-1,3-diene, E-1,1,2,3,4-pentachlorobuta-1,3-diene, and Z-1,1,2,3,4-pentachlorobuta-1,3-diene.

The process may further comprise recovering 2320az from the product mixture prior to use of the recovered 2320az as a starting material in a process to produce E- and Z-1326mxz, 1,1,1,4,4,4-hexafluoro-2-butyne and HFO-Z-1336mzz, for example, as set forth herein.

Processes for recovering 2320az from the product mixture may include one or any combination of purification techniques, such as distillation, that are known in the art. By "recovering" 2320az from the product mixture, a product comprising at least 95% or at least 97% or at least 99% 2320az is produced.

In certain embodiments, the process to produce 2320az may further comprise recovering trichloroethylene from the product mixture and recycling the recovered trichloroethylene to the dimerization process as set forth herein.

In certain embodiments, the process to produce 2320az may further comprise recovering hexachlorobutene isomers from the product mixture and recycling the recovered hexachlorobutene isomers to the dimerization process as set forth herein.

In certain embodiments, the process to produce 2320az may further comprise recovering pentachloroethane from the product mixture and recycling the recovered pentachloroethane to the dimerization process as set forth herein.

Other products, if present, such as E-1,1,2,3,4-pentachloro-1,3-butadiene and Z-1,1,2,3,4-pentachloro-1,3-butadiene may also be recovered.

### Production of E- and Z-1,1,1,4,4,4-hexafluoro-2-chloro-2-butene (E/Z-1326mxz)

There is provided herein a fluorination process comprising contacting 1,1,2,4,4-pentachlorobuta-1,3-diene (2320az) with HF, in the presence of a fluorination catalyst comprising a metal halide and a chlorine source, to provide a product mixture comprising E- and Z-1,1,1,4,4,4-hexafluoro-2-chloro-2-butene.

Fluorination catalyst comprises at least one metal halide, metal oxide or metal oxyhalide. This reaction is performed in the vapor phase. The process produces a product mixture comprising E- and Z-1,1,1,4,4,4-hexafluoro-2-chloro-2-butene.

As used herein, the term "halide" refers to fluorides, chlorides, and bromides.

Examples of suitable metals include nickel, chromium, iron, scandium, yttrium, lanthanum, titanium, zirconium, hafnium, vanadium, molybdenum, tungsten, manganese, rhenium, ruthenium, osmium, cobalt, palladium, copper, zinc, tantalum, antimony, aluminum, tin, and lead. It is noted, as defined herein, antimony is a metal.

Examples of metal halides include nickel halides, chromium halides, iron halides, scandium halides, yttrium halides, lanthanum halides, titanium halides, zirconium halides, hafnium halides, vanadium halides, molybdenum halides, tungsten halides, manganese halides, rhenium halides, ruthenium halides, osmium halides, cobalt halides, palladium halides, copper halides, zinc halides, antimony halides, tantalum halides, aluminum halides, tin halides, and lead halides. In an embodiment, the metal halide is nickel halide, iron halide, or chromium halide or combination thereof is used as a catalyst with or without support on activated carbon. In another embodiment, the metal halide is a bromide or chloride. In still another embodiment, the halide is a chloride. In another embodiment, the metal halide is nickel chloride, iron chloride, or chromium chloride or combination thereof.

Examples of metal oxides include chromium oxide, aluminum oxide. Metal oxyhalides may also be used as fluorination catalysts.

The fluorination catalysts may be unsupported or supported on activated carbon. The activated carbon may be unwashed or be acid washed or base washed.

The term "activated carbon" includes any carbon with a relatively high surface area such as from about 50 to about 3000 m² or from about 100 to about 2000 m² (e.g. from about 200 to about 1500 m² or about 300 to about 1000 m²). The activated carbon may be derived from any carbonaceous material, such as coal (e.g. charcoal), nutshells (e.g. coconut) and wood. Any form of activated carbon may be used, such as powdered, granulated and pelleted activated carbon.

In some embodiments, the activated carbon has been washed with at least one basic solution to remove silicates. For example, the activated carbon is washed with alkali hydroxide or alkaline earth hydroxide or ammonium hydroxide. Examples of basic solutions which have been used to wash the activated carbon include sodium hydroxide, ammonium hydroxide, potassium hydroxide, and the like.

The fluorination process is performed in the presence of a chlorine source. The chlorine source may be chosen from (i) a catalyst comprising a metal chloride or a metal oxychloride, such as a catalyst comprising chromium chloride (CrCl₃) either as the metal chloride or as chromium chloride supported on carbon, or (ii) chlorine (Cl₂), which is added to the process when the fluorination catalyst comprises a metal halide or metal oxyhalide wherein the halide is fluoride or bromide or when the catalyst is a metal oxide. Optionally chlorine (Cl₂) is added when the catalyst is a metal chloride or metal oxychloride.

In one embodiment, the fluorination catalyst comprises a metal chloride or metal oxychloride. In another embodiment the fluorination catalyst does not comprise a metal chloride or metal oxychloride and the process is performed in the presence of chlorine (Cl₂). When chlorine (Cl₂) is present, the molar ratio of chlorine (as Cl₂) to 2320az is typically from about 0.5:1 to about 2:1. A preferred molar ratio of chlorine to 2320az is from about 1.1:1 to about 1:1.

The molar ratio of HF to 2320az, HF:2320az, in some embodiments is from about 1:1 to about 35:1. In other embodiments, the molar ratio of HF to 2320az is from about 1:1 to about 25:1. HF may be added in an amount of 10 to 30 moles per mole of 2320az. In some embodiments, the ratio of HF:2320az:Cl₂ is 10-30:1:1.

The process is performed at effective temperatures and pressures. In an embodiment, the process is performed in the vapor phase at a temperature ranging from about 250 to about 425°C. In another embodiment, the process is performed at a temperature ranging from 275 to about 400°C. In still another embodiment, the process is performed at a temperature ranging from about 300 to about 375°C, and in another embodiment from about 325 to about 350°C.

In an embodiment, the process is performed in the vapor phase at a pressure ranging from about 0 to 1380 kPa (0 to 200 psig).

In another embodiment, the pressure ranges from about 207 to 1034 kPa (30 to 150 psig), and in another embodiment, the pressure ranges from about 276 to 552 kPa (40 to 80 psig).

In an embodiment the process is performed at a temperature ranging from about 275 to about 375°C and at a pressure ranging from about 0 psig to about 160 psig, and in another embodiment temperature ranging from about 300 to about 350°C and at a pressure ranging from about 0 psig to about 80 psig.

In a preferred embodiment, 2320az is vaporized, optionally in the presence of HF, and fed to a vapor phase reactor along with HF and Cl₂.

The process may further comprise recovering E/Z-1326mxz from the product mixture prior to use of the recovered E/Z-1326mxz as a starting material in a process to produce 1,1,1,4,4,4-hexafluoro-2-butyne. Processes for recovering E/Z-1326mxz from the product mixture may include one or any combination of purification techniques, such as distillation, that are known in the art. By "recovering" E/Z-1326mxz from the product mixture, a product comprising at least 95% or at least 97% or at least 99% E/Z-1326mxz is produced. There is no need to separate the E- and Z-1326mxz isomers.

The product mixture comprising E- and Z-1,1,1,4,4,4-hexafluoro-2-chloro-2-butene may also comprise E- and/or Z-1,1,1,4,4,4-hexafluoro-2-butene. In the event either E- or Z-1,1,1,4,4,4-hexafluoro-2-butene is present in the product mixture, each may be recovered for use as a product or reactant in another process.

### Production of 1,1,1,4,4,4-hexafluoro-2-butyne

The present disclosure provides processes for dehydrochlorination of E- and Z-1326mxz at temperatures well below 100°C using a basic aqueous solution in combination with quaternary alkylammonium salts as a phase transfer catalyst.

The present disclosure further provides a process comprising contacting *E*- and/or Z-1326mxz with base to produce a product mixture comprising 1,1,1,4,4,4-hexafluoro-2-butyne (CF₃C≡CCF₃) in a dehydrochlorination reaction. The base is preferably a basic aqueous medium. This reaction step is preferably performed in the presence of a catalyst. Preferably the basic aqueous medium comprises a solution of an alkali metal hydroxide or alkali metal halide salt or other base in water. Preferably the catalyst is a phase transfer catalyst.

As used herein, phase transfer catalyst is intended to mean a substance that facilitates the transfer of ionic compounds between an organic phase and an aqueous phase. In this step, the organic phase comprises the E- and/or Z-1326mxz reactant, and the aqueous phase comprises the basic aqueous medium. The phase transfer catalyst facilitates the reaction of these dissimilar and incompatible components.

While various phase transfer catalysts may function in different ways, their mechanism of action is not determinative of their utility in the present invention provided that the phase transfer catalyst facilitates the dehydrochlorination reaction.

A phase transfer catalyst as used herein is a quaternary alkylammonium salt wherein the alkyl groups are alkyl chains having from four to twelve carbon atoms. In one embodiment, the quaternary alkyl ammonium salt is a tetrabutylammonium salt. The anions of the salt can be halides such as chloride or bromide, hydrogen sulfate, or any other commonly used anion.

In some embodiments, at least one alkyl group of the quaternary alkylammonium salt contains at least 8 carbons. An example of quaternary alkylammonium salt wherein three alkyl groups contain at least 8 carbon atoms includes trioctylmethylammonium chloride. Aliquat^{®} 336 is a commercially available phase transfer catalyst which contains trioctylmethylammonium chloride. An example of quaternary alkylammonium salt wherein four alkyl groups contain at least 8 carbon atoms includes tetraoctylammonium salt. The anions of such salts may be halides such as chloride or bromide, hydrogen sulfate, or any other commonly used anion. Specific quaternary alkylammonium salts include tetraoctylammonium chloride, tetraoctylammonium hydrogen sulfate, tetraoctylammonium bromide, methytrioctylammonium chloride, methyltrioctylammonium bromide, tetradecylammonium chloride, tetradecylammonium bromide, and tetradodecylammonium chloride.

Other compounds commonly thought of as phase transfer catalysts in other applications, including crown ethers, cryptands or non-ionic surfactants alone, do not have a significant effect on conversion or the rate of the dehydrochlorination reaction in the same fashion.

The Z- and E- isomers of 1,1,1,4,4,4-hexafluoro-2-chloro-2-butene exhibit significantly different reactivities with respect to dehydrochlorination, and have different requirements for what functions as an effective phase transfer catalyst in this reaction.

Dehydrochlorination of the Z- isomer of 1,1,1,4,4,4-hexafluoro-2-chloro-2-butene, Z-1326mxz, can be effected with quaternary alkylammonium salts wherein the alkyl groups are alkyl chains having from four to twelve carbon atoms. The anions of the salt can be halides such as chloride or bromide, hydrogen sulfate, or any other commonly used anion. In one embodiment, the quaternary alkyl ammonium salt is a tetrabutylammonium salt. In another embodiment, the quaternary alkylammonium salt is a tetrahexylammonium salt. In another embodiment, the quaternary alkylammonium salt is a tetraoctylammonumium salt. In yet another embodiment, the quaternary alkylammonium salt is a trioctylmethylammonumium salt.

Dehydrochlorination of the E-isomer of 1,1,1,4,4,4-hexafluoro-2-chloro-2-butene, E-1326mxz, can be effected with quaternary alkylammonium salts, wherein the alkyl groups are alkyl chains having at least one alkyl chain of 8 carbons or more. In another embodiment, the quaternary alkylammonium salt has three alkyl chains of 8 carbons or more, such as trioctylmethylammonium salt. In yet another embodiment, the quaternary alkylammonium salt is a tetraoctylammonumium salt. In yet another embodiment, the quaternary ammonium salt is a tetradecylammonium salt. In yet another embodiment, the quaternary alkylammonium salt is a tetradodecylammonium salt. The anions of the salt can be halides such as chloride or bromide, hydrogen sulfate, or any other commonly used anion.

In yet another embodiment, dehydrochlorination of E-1326mxz can be effected with quaternary alkylammonium salts, wherein the alkyl groups are alkyl chains having from four to twelve carbon atoms, and in the presence of a non-ionic surfactant. The non-ionic surfactants can be ethoxylated nonylphenols, and ethoxylated C12 to C15 linear aliphatic alcohols. Suitable non-ionic surfactants include Bio-soft^{®} N25-9 and Makon^{®} 10 are from Stepan Company.

In one embodiment, the quaternary alkylammonium salts is added in an amount of from 0.5 mole percent to 2 mole percent of 1326mxz. In another embodiment, the quaternary alkylammonium salts is added in an amount of from 1 mole percent to 2 mole percent of 1326mxz. In yet another embodiment, the quaternary alkylammonium salts is added in an amount of from 1 mole percent to 1.5 mole percent of 1326mxz.

In one embodiment, the dehydrochlorination of Z- or E-1326mxz is conducted in the presence of an alkali metal halide salt. In one embodiment, the alkali metal is sodium or potassium. In one embodiment, the halide is chloride or bromide. In one embodiment, the alkali metal halide salt is sodium chloride. Without wishing to be bound by any particular theory, it is believed that the alkali metal halide salt stabilizes the phase transfer catalyst. Although the dehydrochlorination reaction itself produces alkali metal chloride, and in particular sodium chloride if sodium hydroxide is used as the base, addition of extra sodium chloride provides a further effect of increasing the yield of hexafluoro-2-butyne.

Addition of alkali metal halide salt also reduces the amount of fluoride ion measured in the water effluent from the reaction. Without wishing to be bound by any particular theory, the presence of fluoride is believed to result from decomposition of either 1326mxz starting material, or 1,1,1,4,4,4-hexafluoro-2-butyne product.

In several samples, the amount of fluoride ion found in the water effluent from the dehydrochlorination is about 6000 ppm. In several examples, using from 30 to 60 equivalents of sodium chloride per mole of phase transfer catalyst, the amount of fluoride ion in the water effluent is reduced to 2000 ppm. In one embodiment, the alkali metal halide is added at from 25 to 100 equivalents per mole of phase transfer catalyst. In another embodiment, the alkali metal halide is added at from 30 to 75 equivalents per mole of phase transfer catalyst. In yet another embodiment, the alkali metal halide is added at from 40 to 60 equivalents per mole of phase transfer catalyst.

In one embodiment, the reaction is conducted at a temperature of from about 60 to 90°C. In another embodiment, the reaction is conducted at 70°C.

As used herein, the basic aqueous solution is a liquid (whether a solution, dispersion, emulsion, or suspension and the like) that is primarily an aqueous liquid having a pH of over 7. In some embodiments the basic aqueous solution has a pH of over 8. In some embodiments, the basic aqueous solution has a pH of over 10. In some embodiments, the basic aqueous solution has a pH of 10-13. In some embodiments, the basic aqueous solution contains small amounts of organic liquids which may be miscible or immiscible with water. In some embodiments, the liquid medium in the basic aqueous solution is at least 90% water. In one embodiment the water is tap water; in other embodiments the water is deionized or distilled.

The base in the aqueous basic solution is selected from the group consisting of hydroxide, oxide, carbonate, or phosphate salts of alkali, alkaline earth metals and mixtures thereof. In one embodiment, bases which may be used lithium hydroxide, sodium hydroxide, potassium hydroxide, calcium hydroxide, magnesium oxide, calcium oxide, sodium carbonate, potassium carbonate, trisodium phosphate, disodium hydrogenphosphate, sodium dihydrogen phosphate, tripotassium phosphate, dipotassium hydrogenphosphate, potassium dihydrogen phosphate, and mixtures thereof.

The product 1,1,1,4,4,4-hexafluoro-2-butyne (boiling point -25°C) may be recovered from the product mixture by distillation, wherein the butyne vaporizes from the aqueous medium and can then be condensed. Any unconverted E- and/or Z-1326mxz can be recovered from the organic phase of the product mixture and recycled for the dehydrochlorination process.

### Production of Z-1,1,1,4,4,4-hexafluoro-2-butene

The present disclosure further provides a hydrogenation process comprising contacting 1,1,1,4,4,4-hexafluoro-2-butyne with hydrogen to produce a product mixture comprising *Z*-1,1,1,4,4,4-hexafluoro-2-butene (Z-1336mzz). This process is preferably performed in the presence of an alkyne-to-alkene catalyst.

In some embodiments the hydrogenation of 1,1,1,4,4,4-hexafluoro-2-butyne is performed as a batch process in the liquid phase.

In some embodiments the hydrogenation of 1,1,1,4,4,4-hexafluoro-2-butyne is performed as a continuous process in the vapor phase.

In some embodiments, an alkyne-to-alkene catalyst is a palladium catalyst, such as palladium dispersed on aluminum oxide or titanium silicate, doped with silver and/or a lanthanide. The loading of palladium dispersed on the aluminum oxide or titanium silicate is relatively low. In some embodiments, the palladium loading is from about 100 ppm to about 5000 ppm. In other embodiments, the palladium loading is from about 200 ppm to about 5000 ppm. In some embodiments, the palladium catalyst is doped with at least one of silver, cerium or lanthanum. In some embodiments, the mole ratio of cerium or lanthanum to palladium is from about 2:1 to about 3:1. In some embodiments the mole ratio of silver to palladium is about 0.5:1.0.

Other embodiments of alkyne-to-alkene catalyst is Lindlar catalyst, which is a heterogeneous palladium catalyst on a calcium carbonate support, which has been deactivated or conditioned with a lead compound. The lead compound may be lead acetate, lead oxide, or any other suitable lead compound. In some embodiments, the catalyst is produced by reduction of a palladium salt in the presence of a slurry of calcium carbonate, followed by the addition of the lead compound. In some embodiments, the palladium salt in palladium chloride.

In other embodiments, the Lindlar catalyst is further deactivated or conditioned with quinoline. The amount of palladium on the support is typically about 5% by weight but may be any catalytically effective amount. In other embodiments, the amount of palladium on the support in the Lindlar catalyst is greater than 5% by weight. In yet other embodiments, the amount of palladium on the support may be from about 5% by weight to about 1% by weight.

In some embodiments, the amount of the catalyst used is from about 0.5% by weight to about 4% by weight of the amount of the 1,1,1,4,4,4-hexafluoro-2-butyne. In other embodiments, the amount of the catalyst used is from about 1% by weight to about 3% by weight of the amount of the butyne. In yet other embodiments, the amount of the catalyst used is from about 1% to about 2% by weight of the amount of the butyne.

In some embodiments, this reaction step is a batch reaction and is performed in the presence of a solvent. In one such embodiment, the solvent is an alcohol. Typical alcohol solvents include ethanol, i-propanol and n-propanol. In other embodiments, the solvent is a fluorocarbon or hydrofluorocarbon. Typical fluorocarbons or hydrofluorocarbons include 1,1,1,2,2,3,4,5,5,5-decafluoropentane and 1,1,2,2,3,3,4-heptafluorocyclopentane.

In some embodiments, reaction of the 1,1,1,4,4,4-hexafluoro-2-butyne with hydrogen is preferably performed with addition of hydrogen in portions, with increases in the pressure of the vessel of no more than about 100 psi (0.69 MPa) with each addition. In other embodiments, the addition of hydrogen is controlled so that the pressure in the vessel increases no more than about 50 psi (0.35 MPa) with each addition. In some embodiments, after enough hydrogen has been consumed in the hydrogenation reaction to convert at least 50% of the butyne to Z-1336mzz, hydrogen may be added in larger increments for the remainder of the reaction. In other embodiments, after enough hydrogen has been consumed in the hydrogenation reaction to convert at least 60% of the butyne to the desired butene, hydrogen may be added in larger increments for the remainder of the reaction. In yet other embodiments, after enough hydrogen has been consumed in the hydrogenation reaction to convert at least 70% of the butyne to desired butene, hydrogen may be added in larger increments for the remainder of the reaction. In some embodiments, the larger increments of hydrogen addition may be 300 psi (2.07 MPa). In other embodiments, the larger increments of hydrogen addition may be 400 psi (2.76 MPa).

In some embodiments, the molar ratio is about 1 mole of hydrogen to about 1 mole of 1,1,1,4,4,4-hexafluoro-2-butyne. In other embodiments, the molar ratio is from about 0.9 mole to about 1.3 mole, hydrogen to butyne. In yet other embodiments, the amount of hydrogen added is from about 0.95 mole of hydrogen to about 1.1 moles of butyne. In yet other embodiments, the amount of hydrogen added is from about 0.95 moles of hydrogen to about 1.03 moles of butyne.

In some embodiments, the hydrogenation is performed at ambient temperature (15°C to 25°C). In other embodiments, the hydrogenation is performed at above ambient temperature. In yet other embodiments, the hydrogenation is performed at below ambient temperature. In yet other embodiments, the hydrogenation is performed at a temperature of below about 0°C.

In an embodiment of a continuous process, a mixture of 1,1,1,4,4,4-hexafluoro-2-butyne and hydrogen is passed through a reaction zone containing the catalyst. A reaction vessel, e.g., a metal tube, may be used, packed with the catalyst to form the reaction zone. In some embodiments, the molar ratio of hydrogen to the butyne is about 1:1. In other embodiments of a continuous process, the molar ratio of hydrogen to the butyne is less than 1:1. In yet other embodiments, the molar ratio of hydrogen to the butyne is about 0.67:1.0.

In some embodiments of a continuous process, the reaction zone is maintained at ambient temperature. In other embodiments of a continuous process, the reaction zone is maintained at a temperature of 30°C. In yet other embodiments of a continuous process, the reaction zone is maintained at a temperature of about 40°C.

In some embodiments of a continuous process, the flow rate of 1,1,1,4,4,4-hexafluoro-2-butyne and hydrogen is maintained so as to provide a residence time in the reaction zone of about 30 seconds. In other embodiments of a continuous process, the flow rate of the butyne and hydrogen is maintained so as to provide a residence time in the reaction zone of about 15 seconds. In yet other embodiments of a continuous process, the flow rate of butyne and hydrogen is maintained so as to provide a residence time in the reaction zone of about 7 seconds.

It will be understood, that residence time in the reaction zone is reduced by increasing the flow rate of 1,1,1,4,4,4-hexafluoro-2-butyne and hydrogen into the reaction zone. As the flow rate is increased this will increase the amount of butyne being hydrogenated per unit time. Since the hydrogenation is exothermic, depending on the length and diameter of the reaction zone, and its ability to dissipate heat, at higher flow rates it may be desirable to provide a source of external cooling to the reaction zone to maintain a desired temperature.

The conditions of the contacting step, including the choice of catalyst, are preferably selected to produce Z-1336mzz at a selectivity of at least 85%, more preferably at least 90%, and most preferably at least 95%.

In some embodiments, upon completion of a batch-wise or continuous hydrogenation process, the Z-1336mzz may be recovered through any conventional process, including for example, fractional distillation. Unconverted hexafluoro-2-butyne may be recovered and recycled to the hydrogenation process. In other embodiments, upon completion of a batch-wise or continuous hydrogenation process, the Z-1336mzz is of sufficient purity to not require further purification steps.

### EXAMPLES

### Materials

Trichloroethylene, ferric chloride, chromium chloride, alumina chloride, cupric chloride, chlorine, pentachloroethane (HCC-120), trioctylmethylammonium chloride (Aliquat^{®} 336), NaOH, K₂HPO₄ and KH₂PO₄, and Lindlar catalyst are available from Sigma Aldrich, St. Louis, MO. Hydrogen fluoride and E-1,1,1,4,4,4-hexafluoro-2-butene are available from Synquest Labs, Inc., Alachua, FL. 10% chrome chloride on carbon catalyst is available from BASF, Iselin, NJ.

GC analysis for Examples 1-4 was performed using Agilent^{®} 5975GC, RESTEK Rtx-1 column.

### Example 1: Preparation of 1,1,2,4,4-pentachlorobuta-1,3-diene (HCC-2320az)

Trichloroethylene (100 g, 0.76 mol) was added to a shaker tube containing 30 mg anhydrous FeCl₃. The reaction mixture was heated at 230°C for 2 hrs. The reactor content was cooled to room temperature and analyzed by GC to determine the conversion and selectivity. Results are provided in Table 1.

### Example 2: Preparation of 1,1,2,4,4-pentachlorobuta-1,3-diene (HCC-2320az)

Trichloroethylene (100 g, 0.76 mol) was added to a shaker tube containing 1 g iron wire. The reaction mixture was heated at 230°C for 2 hrs. The reactor content was cooled to room temperature and analyzed by GC to determine the conversion and selectivity. Results are provided in Table 1.

### Example 3: Preparation of 1,1,2,4,4-pentachlorobuta-1,3-diene (HCC-2320az)

Trichloroethylene (100 g, 0.76 mol) was added to a shaker tube containing 20 mg anhydrous FeCl₃ and 1 g HCC-120. The reaction mixture was heated at 230°C for 2 hrs. The reactor content was cooled to room temperature and analyzed by GC to determine the conversion and selectivity. Results are provided in Table 1.

### Example 4: Preparation of 1,1,2,4,4-pentachlorobuta-1,3-diene (HCC-2320az)

Trichloroethylene (100 g, 0.76 mol) was added to a shaker tube containing 1 g iron wire and 1 g HCC-120. The reaction mixture was heated at 230°C for 2 hrs. The reactor content was cooled to room temperature and analyzed by GC to determine the conversion and selectivity. Results are provided in Table 1.

**Table 1. Trichloroethylene Dimerization to 2320az**

| **Example** | **Catalyst** | **Time (hours)** | **Conversion /Selectivity (%)** |
|---|---|---|---|
| 1 | FeCl₃ (30 mg) | 16 | 26.9 / 81.6 |
| 2 | Fe wire (1 g) | 8 | 28.0 / 86.7 |
| 3 | FeCl₃ (20 mg) / HCC-120 (1 g) | 2 | 35.4 / 84.3 |
| 4 | Fe wire (1 g) / HCC-120 (1 g) | 2 | 32.3 / 87.4 |

As can be seen from Table 1, the presence of HCC-120 increases conversion rate of trichloroethylene to 2320az when using FeCl₃ or Fe wire catalyst.

### Example 5: Preparation of E- and Z-1,1,1,4,4,4-hexafluoro-2-chloro-2-butene

An Inconel tube^{®} of 1,27 cm (0,5 inch) OD, 38,1 cm (15 inch) length and 0,86 cm (0,34 inch) wall thinkness was filled with 12 cc (6.45 g) of 10% chrome chloride on carbon catalyst. The reactor was heated in a Lindberg furnace to 250°C and 2320az was fed at 0.09 ml/hour and HF gas at 5.4 sccm (standard cubic centimeters per minute) through a vaporizer controlled at 200°C. Over the course of the run, the temperature was raised to 325°C. All of the experiments were carried out at 1,3-2,7 kPa (1-2 psig).

The effluent of the reactor is analyzed online using an Agilent^{®} 6890 GC/5973 MS and a Restek^{®} PC2618 5% Krytox^{®} CBK-D/60/80 6 meter x 2mm ID 0,32 cm (1/8") OD packed column purged with helium at 30 sccm. Run conditions are provided in Table 2 (1 psi ₌ 6895 Pa) Data is shown in Table 3, and samples are taken in hourly intervals.

**Table 2: Run Conditions for Vapor Phase Fluorination of 2320az**

| Sample No. | Furnace Temp., °C | Pressure, psi | Liquid, ml//hr | N₂, sccm | HF, sccm | Contact Time, sec |
|---|---|---|---|---|---|---|
| 1 | 249 | 1.4 | 0.09 | 3.13 | 5.40 | 51.2 |
| 2 | 250 | 1.4 | 0.09 | 3.10 | 5.40 | 51.3 |
| 3 | 250 | 1.3 | 0.09 | 3.13 | 5.40 | 50.8 |
| 4 | 250 | 1.3 | 0.09 | 3.13 | 5.40 | 50.8 |
| 5 | 250 | 1.0 | 0.09 | 3.09 | 5.40 | 50.1 |
| 6 | 275 | 0.9 | 0.09 | 3.10 | 5.40 | 47.4 |
| 7 | 275 | 0.9 | 0.09 | 3.05 | 5.40 | 47.7 |
| 8 | 275 | 0.9 | 0.09 | 3.10 | 5.40 | 47.4 |
| 9 | 300 | 1.0 | 0.09 | 3.10 | 5.40 | 45.7 |
| 10 | 300 | 1.0 | 0.09 | 3.10 | 5.40 | 45.6 |
| 11 | 300 | 1.0 | 0.09 | 3.14 | 5.40 | 45.4 |
| 12 | 325 | 0.9 | 0.09 | 3.09 | 5.40 | 43.5 |
| 13 | 325 | 1.0 | 0.09 | 3.09 | 5.40 | 43.8 |
| 14 | 325 | 1.0 | 0.09 | 3.07 | 5.40 | 43.9 |

**Table 3: Products from Vapor Phase Fluorination of 2320az (expressed as mole%)**

| Sample No. | Z-1326mxz | E-1326mxz | Z-1336mzz | E-1336mzz | 1316mxx | 1325 | 1327 | 338mff | 346mdf | 356mff | Unknowns |
|---|---|---|---|---|---|---|---|---|---|---|---|
| 1 | 64.23 | 2.12 | 0.00 | 24.90 | 0.00 | 0.00 | 0.00 | 7.56 | 0.00 | 0.00 | 1.20 |
| 2 | 75.98 | 2.41 | 0.00 | 13.58 | 0.00 | 0.00 | 0.00 | 6.83 | 0.00 | 0.00 | 1.20 |
| 3 | 61.01 | 1.69 | 0.00 | 31.68 | 0.00 | 0.00 | 0.00 | 1.17 | 0.00 | 0.00 | 4.45 |
| 4 | 84.26 | 2.24 | 0.41 | 7.86 | 0.00 | 0.00 | 0.00 | 0.48 | 0.00 | 0.88 | 3.88 |
| 5 | 80.58 | 4.09 | 0.46 | 0.00 | 0.00 | 0.00 | 6.86 | 0.56 | 0.00 | 0.89 | 6.56 |
| 6 | 83.34 | 3.32 | 0.00 | 3.88 | 0.15 | 1.51 | 3.99 | 0.16 | 0.00 | 0.00 | 3.65 |
| 7 | 81.96 | 3.23 | 0.00 | 4.68 | 0.23 | 3.55 | 3.30 | 0.28 | 0.00 | 0.00 | 2.77 |
| 8 | 78.64 | 3.05 | 0.00 | 5.53 | 0.48 | 5.51 | 3.37 | 0.53 | 0.00 | 0.00 | 2.88 |
| 9 | 77.76 | 3.40 | 0.00 | 7.42 | 0.61 | 3.47 | 2.65 | 0.92 | 0.00 | 0.00 | 3.77 |
| 10 | 73.08 | 3.26 | 0.00 | 13.93 | 0.97 | 1.89 | 0.00 | 2.30 | 0.00 | 0.00 | 4.58 |
| 11 | 70.58 | 3.02 | 0.00 | 12.59 | 1.12 | 1.77 | 3.72 | 2.71 | 0.00 | 0.00 | 4.49 |
| 12 | 66.90 | 3.39 | 0.00 | 19.45 | 1.27 | 0.25 | 0.00 | 3.80 | 0.00 | 0.00 | 4.94 |
| 13 | 67.00 | 3.10 | 0.00 | 17.85 | 0.94 | 0.00 | 3.84 | 3.80 | 0.00 | 0.00 | 3.47 |
| 14 | 63.82 | 2.97 | 0.00 | 24.12 | 0.96 | 0.00 | 0.00 | 4.49 | 0.00 | 0.00 | 3.64 |

### Example 6: Preparation of 1,1,1,4,4,4-Hexafluoro-2-butyne

NaOH aqueous solution (12 mL, 0.12 mol) was added to the mixture of Z-1326 (20 g, 0.1 mol) and water (18 mL) in the presence of tetra-n-butylammonium bromide (0.45 g, 0.001325 mol) at 35°C. The reaction temperature was raised to 70°C after the addition, and gas chromatography was used to monitor the reaction. The reaction was completed after 1 hour and 15.4 g product (conversion: 100%; yield: 95%) was collected in a dry ice trap. Results are provided in Table 4.

### Example 7: Preparation of 1,1,1,4,4,4-Hexafluoro-2-butyne

NaOH aqueous solution (12 mL, 0.12 mol) was added to the mixture of Z-1326mxz (20 g, 0.1 mol) and water (18 mL) in the presence of tetra-n-butylammonium hydrogensulfate (0.43 g, 0.001325 mol) at 35°C. The reaction temperature was raised to 70°C after the addition, and gas chromatography was used to monitor the reaction. The reaction was completed after 1 hour and 11 product (conversion: 100%; yield: 71%) was collected in a dry ice trap. Results are provided in Table 4.

### Example 8: Preparation of 1,1,1,4,4,4-Hexafluoro-2-butyne

NaOH aqueous solution (12 mL, 0.12 mol) was added to the mixture of Z-1326mxz (20 g, 0.1 mol) and water (18 mL) in the presence of Aliquat^{®} 336 (0.53 g, 0.001325 mol) at 35°C. The reaction temperature was raised to 70°C after the addition, and gas chromatography was used to monitor the reaction. The reaction was completed after 1 hour and 15.6 product (conversion: 100%; yield: 96%) was collected in a dry ice trap. Results are provided in Table 4.

### Example 9: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of Aliquat^{®} 336 (0.53 g, 0.001325 mol) at 42°C. The reaction temperature was raised to 70°C after the addition, and gas chromatography was used to monitor the reaction. The reaction was completed after 1 hours and 15.8 g product (conversion: 100%; yield: 98%) was collected in a dry ice trap. Results are provided in Table 4.

### Example 10: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tetra-n-butylammonium bromide (0.45 g, 0.001325 mol) at 42°C. The reaction temperature was raised to 70°C after the addition, and gas chromatography was used to monitor the reaction. The reaction was not completed after seven hours. 12.6 g product (conversion: 78%; yield: 78%) was collected in a dry ice trap. Results are provided in Table 4.

### Example 11: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tetra-n-butylammonium hydrogen sulfate (0.43 g, 0.001325 mol) at 42°C. The reaction temperature was raised to 70°C after the addition, and gas chromatography was used to monitor the reaction. The reaction was not completed after seven hours. 12.6 g product (conversion: 77%; yield: 77%) was collected in a dry ice trap. Results are provided in Table 4.

### Example 12: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tetraoctylammonium bromide (0.72 g, 0.001325 mol) at 42°C. The reaction temperature was raised to 70°C after the addition, and gas chromatography was used to monitor the reaction. The reaction was completed after six and half hours. 15.6 g product (conversion: 100%; yield: 95%) was collected in a dry ice trap. Results are provided in Table 4.

### Example 13: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tetraoctylammonium chloride (0.43 g, 0.001325 mol) at 42°C. The reaction temperature was raised to 70°C after the addition, and gas chromatography was used to monitor the reaction. After five and half hours, 15.2 g product (conversion: 95%; yield: 93%) was collected in a dry ice trap. Results are provided in Table 4.

### Example 14: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tetra-n-butylammonium chloride (0.37 g, 0.001325 mol) at 42°C. The reaction temperature was raised to 70°C after the addition, and gas chromatography was used to monitor the reaction. After twenty-three hours, 14.8 g product (conversion: 90%; yield: 87%) was collected in a dry ice trap. Results are provided in Table 4.

### Example 15: Preparation of 1,1,1,4,4,4-Hexafluoro-2-butyne

NaOH aqueous solution (12 mL, 0.12 mol) was added to the mixture of E-1326mxz (20 g, 0.1 mol) and water (18 mL) in the presence of tributylmethylammonium chloride (0.31 g, 0.001325 mol) at 42°C. The reaction temperature was raised to 70°C after the addition, and gas chromatography was used to monitor the reaction. After twenty-three hours, 8 g product (conversion: 59%; yield: 49%) was collected in a dry ice trap. Results are provided in Table 4.

### Example 16: Preparation of 1,1,1,4,4,4-Hexafluoro-2-butyne

NaOH aqueous solution (12 mL, 0.12 mol) was added to the mixture of *ZE-*1326mxz (a mixture of 50% Z-1326mxz and 50% E-1326mxz) (20 g, 0.1 mol) and water (18 mL) in the presence of tetrabutylammonium bromide (0.45 g, 0.001325 mol) and Bio-soft^{®} N25-9 (0.7 g) at 38°C. The reaction temperature was raised to 70°C after the addition, and gas chromatography was used to monitor the reaction. The reaction was completed after 5 hours. 13 g product (conversion: 100%; yield: 80%) was collected in a dry ice trap. Results are provided in Table 4.

### Example 17: Preparation of 1,1,1,4,4,4-Hexafluoro-2-butyne

NaOH aqueous solution (12 mL, 0.12 mol) was added to the mixture of *ZE-*1326mxz (a mixture of 50% Z-1326mxz and 50% E-1326mxz) (20 g, 0.1 mol) and water (18 mL) in the presence of tetrabutylammonium bromide (0.45 g, 0.001325 mol) and Makon^{®} 10 (0.7 g) at 38°C. The reaction temperature was raised to 70°C after the addition, and gas chromatography was used to monitor the reaction. The reaction was completed after 5 hours. 11.2 g product (conversion: 100%; yield: 69%) was collected in a dry ice trap. Results are provided in Table 4.

### Example 18: Preparation of 1,1,1,4,4,4-Hexafluoro-2-butyne

10 M NaOH aqueous solution (12 mL, 0.12 mol) was added over 30 min to a ZE-1326mxz (a mixture of 50% Z-1326mxz and 50% E-1326mxz) (20 g, 0.1 mol) and water (18 mL) in the presence of NaCl (2.3 g, 0.0393 mol) and Aliquat^{®} 336 (0.53 g, 0.001325 mol) at 37°C. When the addition was complete, the reaction temperature was raised to 70°C after the addition, and gas chromatography was used to monitor the reaction. The reaction was completed after 1 hour and 20 minutes and the water layer was submitted for wt% fluoride analysis. Results are provided in Table 4.

### Example 19: Preparation of 1,1,1,4,4,4-Hexafluoro-2-butyne

NaOH aqueous solution (12 mL, 0.12 mol) was added over 30 min to a ZE-1326mxz (a mixture of 50% Z-1326mxz and 50% E-1326mxz) (20 g, 0.1 mol) and water (18 mL) in the presence of NaCl (4.6 g, 0.0786 mol) and Aliquat^{®} 336 (0.53 g, 0.001325 mol) at 37°C. When the addition was complete, the reaction temperature was raised to 70°C after the addition, and gas chromatography was used to monitor the reaction. The reaction was completed after 1 hour and 20 minutes and the water layer was submitted for wt% fluoride analysis. Results are provided in Table 4.

### Example 20: Preparation of 1,1,1,4,4,4-Hexafluoro-2-butyne

NaOH aqueous solution (12 mL, 0.12 mol) was added over 30 min to a mixture of ZE-1326mxz (a mixture of 50% Z-1326mxz and 50% E-1326mxz) (20 g, 0.1 mol) and water (18 mL) in the presence of NaCl (3.45 g, 0.0590 mol) and Aliquat^{®} 336 (0.53 g, 0.001325 mol) at 37°C. When the addition was complete, the reaction temperature was raised to 70°C after the addition, and gas chromatography was used to monitor the reaction. The reaction was completed after 2 hours and the water layer was submitted for wt% fluoride analysis. Results are provided in Table 4.

### Comparative Example A

NaOH aqueous solution (12 mL, 0.12 mol) was added to the mixture of ZE-1326mxz (a mixture of 50% Z-1326mxz and 50% E-1326mxz) (20 g, 0.1 mol) and water (18 mL) at 37°C. The reaction temperature was raised to 70°C after the addition, and gas chromatography was used to monitor the reaction. After thirty-one hours. 0.36 g product (conversion: 2.2%; yield: 2.2%) was collected in a dry ice trap. Results are provided in Table 4.

### Comparative Example B

NaOH aqueous solution (6 mL, 0.06 mol) was added to the mixture of ZE-1326mxz (a mixture of 50% Z-1326mxz and 50% E-1326mxz) (10 g, 0.05 mol) and water (18 mL) at 37°C in the presence of 15-Crown-5 (0.65 g, 0.003 mol). The reaction temperature was raised to 70°C after the addition, and gas chromatography was used to monitor the reaction. The reaction was not completed after thirty hours. 1.16 g product (conversion: 14%; yield: 14%) was collected in a dry ice trap. Results are provided in Table 4.

### Comparative Example C

NaOH aqueous solution (12 mL, 0.12 mol) was added to the mixture of ZE-1326mxz (a mixture of 50% Z-1326mxz and 50% E-1326mxz) (20 g, 0.1 mol) and water (18 mL) at 37°C in the presence of Makon^{®} 10 (0.7 g). The reaction temperature was raised to 70°C after the addition, and gas chromatography was used to monitor the reaction. The reaction was not completed after twenty-two hours. 1.09 g product (conversion: 17%; yield: 6.8%) was collected in a dry ice trap. Results are provided in Table 4.

**Table 4. Results of Examples 6-20, Comparative Examples A-C**

| **Example** | **1326** | **Time (hr)** | **Conv. (%)** | **Yield (%)** |
|---|---|---|---|---|
| 6 | Z | 1 | 100 | 100 |
| 7 | Z | 1 | 100 | 100 |
| 8 | Z | 1 | 100 | 100 |
| 9 | E | 1 | 100 | 100 |
| 10 | E | 7 | 78 | 11.1 |
| 11 | E | 6.5 | 77 | 11 |
| 12 | E | 6.5 | 100 | 15.4 |
| 13 | E | 5.5 | 95 | 17.3 |
| 14 | E | 23 | 90 | 3.9 |
| 15 | E | 23 | 59 | 2.6 |
| 16 | ZE | 5 | 100 | 20 |
| 17 | ZE | 4 | 100 | 25 |
| 18 | ZE | 1.3 | 100 | 75 |
| 19 | ZE | 1.3 | 100 | 75 |
| 20 | ZE | 2 | 100 | 50 |
| Comp. A | ZE | 21 | 2.2 | 0.07 |
| Comp. B | ZE | 30 | 14 | 0.47 |
| Comp. C | ZE | 22 | 17 | 0.77 |

Notes: Time is in hours; Conv. refers to weight% conversion of 1326; Yield is weight% yield of 1,1,1,4,4,4-hexafluoro-2-butyne produced.

### Example 21: Preparation of Z-1,1,1,4,4,4-hexafluoro-2-butene

1,1,1,4,4,4-Hexafluoro-2-butyne produced according to Example 9 was reacted with hydrogen to produce the desired Z-isomer of 1,1,1,4,4,4-hexafluoro-2-butene by the following procedure: 5g of Lindlar (5% Pd on CaCOs poisoned with lead) catalyst was charged in 1.3 L rocker bomb. 480g (2.96 mole) of hexafluoro-2-butyne was charged in the rocker. The reactor was cooled (-78°C) and evacuated. After the bomb was warmed to room temperature, H₂ was added slowly, by increments which did not exceed Δp= 50 psi (0.35 MPa). A total of 3 moles H₂ were added to the reactor. A gas chromatographic analysis of the crude product indicated the mixture consisted of CF₃C≡CCF₃ (0.236%), trans-isomer *E*-CF₃CH=CHCF₃ (0.444%), saturated CF₃CH₂CH₂CF₃ (1.9%) CF₂=CHCl, impurity from starting butyne, (0.628%), cis-isomer *Z*-CF₃CH=CHCF₃ (96.748%).

Distillation of the crude product afforded 287g (59%yield) of 100% pure cis-CF₃CH=CHCF₃ (boiling point 33.3°C). MS: 164 [MI], 145 [M-19], 95 [CF₃CH=CH], 69 [CF₃]. NMR ¹H: 6.12 ppm (multiplet), ¹⁹F: -60.9 ppm (triplet J=0.86Hz). The selectivity of this reaction to the formation of the Z-isomer was 96.98%. The Z-isomer was recovered by distillation.

It is to be understood that while the invention has been described in conjunction with the detailed description thereof, the foregoing description is intended to illustrate and not limit the scope of the invention, which is defined by the scope of the appended claims.

## Claims

1. A process for producing a product mixture comprising E- and Z-1,1,1,4,4,4-hexafluoro-2-chloro-2-butene comprising contacting 1,1,2,4,4-pentachlorobuta-1,3-diene with HF in the vapor phase in the presence of a chlorine source and a fluorination catalyst and to produce a product mixture comprising E- and Z-1,1,1,4,4,4-hexafluoro-2-chloro-2-butene.

2. The process of claim 1 wherein the fluorination catalyst comprises a metal chloride or metal oxychloride where the catalyst is the chlorine source.

3. The process of claim 1 wherein the fluorination catalyst comprises a metal halide or metal oxyhalide wherein the halide is not chloride or metal oxide and the chlorine source is chlorine (Cl₂) preferably
wherein the ratio of chlorine to 1,1,2,4,4-pentachlorobuta-1,3-diene is 1 to 10 (molar ratio).

4. The process of claim 1 wherein the process is performed in the presence of chlorine (Cl₂), or
wherein the molar ratio of HF to 1,1,2,4,4-pentachlorobuta-1,3-diene is from 1 to 35, or
wherein the process is performed at a temperature in the range of 250 to 425°C, or
wherein the process is performed at a pressure in the range of 0 to 200 psi (0 to 1.4 MPa).

5. The process of claim 1 further comprising producing 1,1,2,4,4-pentachlorobuta-1,3-diene by contacting trichloroethylene with a dimerization catalyst comprising iron to produce a product mixture comprising 1,1,2,4,4-pentachlorobuta-1,3-diene.

6. The process of claim 5 wherein trichloroethylene is contacted with a dimerization catalyst comprising iron and pentachloroethane.

7. A process for producing a product mixture comprising E- and Z-1,1,1,4,4,4-hexafluoro-2-chloro-2-butene comprising:
(a) producing 1,1,2,4,4-pentachlorobuta-1,3-diene by contacting trichloroethylene with a dimerization catalyst to produce a product mixture comprising 1,1,2,4,4-pentachlorobuta-1,3-diene; and
(b) contacting 1,1,2,4,4-pentachlorobuta-1,3-diene with HF in the vapor phase in the presence of a fluorination catalyst comprising a metal halide to produce a product mixture comprising E- and Z-1,1,1,4,4,4-hexafluoro-2-chloro-2-butene.

8. A process for producing a product mixture comprising E- and Z-1,1,1,4,4,4-hexafluoro-2-chloro-2-butene comprising:
(a) producing 1,1,2,4,4-pentachlorobuta-1,3-diene by contacting trichloroethylene with a dimerization catalyst and pentachloroethane to produce a product mixture comprising 1,1,2,4,4-pentachlorobuta-1,3-diene; and
(b) contacting 1,1,2,4,4-pentachlorobuta-1,3-diene with HF in the vapor phase in the presence of a fluorination catalyst comprising a metal halide to produce a product mixture comprising E- and Z-1,1,1,4,4,4-hexafluoro-2-chloro-2-butene.

9. The process of claim 7 or 8 further comprising recovering 1,1,2,4,4-pentachlorobuta-1,3-diene from the product mixture of step (a), or
further comprising recovering trichloroethylene from the product mixture of step (a).

10. A process to produce Z-1,1,1,4,4,4-hexafluorobut-2-ene, comprising:
(a) contacting trichloroethylene with a dimerization catalyst to produce a product mixture comprising 1,1,2,4,4-pentachlorobuta-1,3-diene;
(b) contacting 1,1,2,4,4-pentachlorobuta-1,3-diene with HF in the vapor phase in the presence of a fluorination catalyst comprising a metal halide to produce a product mixture comprising E- and Z-1,1,1,4,4,4-hexafluoro-2-chloro-2-butene;
(c) contacting E- and/or Z-1,1,1,4,4,4- hexafluoro-2-chloro-2-butene with base to produce a product mixture comprising 1,1,1,4,4,4-hexafluoro-2-butyne; and
(d) contacting 1,1,1,4,4,4-hexafluoro-2-butyne with H₂ to produce a product mixture comprising Z-1,1,1,4,4,4-hexafluorobut-2-ene.

11. The process of 10 further comprising recovering 1,1,2,4,4-pentachlorobuta-1,3-diene from the product mixture of step (a).

12. The process of claim 10 or 11 further comprising recovering trichloroethylene from the product mixture of step (a).

13. The process of any of claims 10, 11, or 12 further comprising recovering E- and Z-1,1,1,4,4,4-hexafluoro-2-chloro-2-butene from the product mixture of step (b).

14. The process of any of claims 10, 11, 12 or 13 further comprising recovering 1,1,1,4,4,4-hexafluoro-2-butyne from the product mixture of step (c).

15. The process of any of claims 10, 11, 12, 13, or 14 further comprising recovering Z-1,1,1,4,4,4-hexafluoro-2-butene from the product mixture of step (d).

## Patentansprüche

1. Verfahren zur Herstellung eines E- und Z-1,1,1,4,4,4-Hexafluor-2-chlor-2-buten umfassenden Produktgemisches, umfassend Inkontaktbringen von 1,1,2,4,4-Pentachlorbuta-1,3-dien mit HF in der Dampfphase in Gegenwart einer Chlorquelle und eines Fluorierungskatalysators und zur Herstellung eines E- und Z-1,1,1,4,4,4-Hexafluor-2-chlor-2-buten umfassenden Produktgemisches.

2. Verfahren nach Anspruch 1, wobei der Fluorierungskatalysator ein Metallchlorid oder Metalloxychlorid umfasst, wobei der Katalysator die Chlorquelle ist.

3. Verfahren nach Anspruch 1, wobei der Fluorierungskatalysator ein Metallhalogenid oder Metalloxyhalogenid umfasst, wobei das Halogenid kein Chlorid oder Metalloxid ist und die Chlorquelle Chlor (Cl₂) ist, vorzugsweise
wobei das Verhältnis von Chlor zu 1,1,2,4,4-Pentachlorbuta-1,3-dien 1 zu 10 (Molverhältnis) beträgt.

4. Verfahren nach Anspruch 1, wobei das Verfahren in Gegenwart von Chlor (Cl₂) durchgeführt wird, oder
wobei das Molverhältnis von HF zu 1,1,2,4,4-Pentachlorbutadien-1,3 von 1 bis 35 beträgt, oder
wobei das Verfahren bei einer Temperatur im Bereich von 250 bis 425 °C durchgeführt wird, oder
wobei das Verfahren bei einem Druck im Bereich von 0 bis 200 psi (0 bis 1,4 MPa) durchgeführt wird.

5. Verfahren nach Anspruch 1, ferner umfassend die Herstellung von 1,1,2,4,4-Pentachlorbuta-1,3-dien durch Inkontaktbringen von Trichlorethylen mit einem Dimerisierungskatalysator, der Eisen umfasst, um ein Produktgemisch herzustellen, das 1,1,2,4,4-Pentachlorbuta-1,3-dien umfasst.

6. Verfahren nach Anspruch 5, wobei Trichlorethylen mit einem Dimerisierungskatalysator, der Eisen umfasst, und Pentachlorethan in Kontakt gebracht wird.

7. Verfahren zur Herstellung eines Produktgemisches, das E- und Z-1,1,1,4,4,4-Hexafluor-2-chlor-2-buten umfasst, umfassend:
(a) Herstellen von 1,1,2,4,4-Pentachlorbuta-1,3-dien durch Inkontaktbringen von Trichlorethylen mit einem Dimerisierungskatalysator, um ein Produktgemisch herzustellen, das 1,1,2,4,4-Pentachlorbuta-1,3-dien umfasst, und
(b) Inkontaktbringen von 1,1,2,4,4-Pentachlorbuta-1,3-dien mit HF in der Dampfphase in Gegenwart eines Fluorierungskatalysators, der ein Metallhalogenid umfasst, um ein Produktgemisch herzustellen, das E- und Z-1,1,1,4,4,4-Hexafluor-2-chlor-2-buten umfasst.

8. Verfahren zur Herstellung eines Produktgemisches, das E- und Z-1,1,1,4,4,4-Hexafluor-2-chlor-2-buten umfasst, umfassend:
(a) Herstellen von 1,1,2,4,4-Pentachlorbuta-1,3-dien durch Inkontaktbringen von Trichlorethylen mit einem Dimerisierungskatalysator und Pentachlorethan, um ein Produktgemisch herzustellen, das 1,1,2,4,4-Pentachlorbuta-1,3-dien umfasst, und
(b) Inkontaktbringen von 1,1,2,4,4-Pentachlorbuta-1,3-dien mit HF in der Dampfphase in Gegenwart eines Fluorierungskatalysators, der ein Metallhalogenid umfasst, um ein Produktgemisch herzustellen, das E- und Z-1,1,1,4,4,4-Hexafluor-2-chlor-2-buten umfasst.

9. Verfahren nach Anspruch 7 oder 8, ferner umfassend die Gewinnung von 1,1,2,4,4-Pentachlorbuta-1,3-dien aus dem Produktgemisch von Schritt (a), oder
ferner umfassend die Gewinnung von Trichlorethylen aus dem Produktgemisch von Schritt (a).

10. Verfahren zur Herstellung von Z-1,1,1,4,4,4-Hexafluorbut-2-en, umfassend:
(a) Inkontaktbringen von Trichlorethylen mit einem Dimerisierungskatalysator, um ein Produktgemisch herzustellen, das 1,1,2,4,4-Pentachlorbut-1,3-dien umfasst,
(b) Inkontaktbringen von 1,1,2,4,4-Pentachlorbuta-1,3-dien mit HF in der Dampfphase in Gegenwart eines Fluorierungskatalysators, der ein Metallhalogenid umfasst, um ein Produktgemisch herzustellen, das E- und Z-1,1,1,4,4,4-Hexafluor-2-chlor-2-buten umfasst,
(c) Inkontaktbringen von E- und/oder Z-1,1,1,4,4,4-Hexafluor-2-chlor-2-buten mit einer Base, um ein Produktgemisch herzustellen, das 1,1,1,4,4,4-Hexafluor-2-butin umfasst, und
(d) Inkontaktbringen von 1,1,1,4,4,4-Hexafluor-2-butin mit H₂, um ein Produktgemisch herzustellen, das Z-1,1,1,4,4,4-Hexafluorbut-2-en umfasst.

11. Verfahren nach Anspruch 10, ferner umfassend die Gewinnung von 1,1,2,4,4-Pentachlorbuta-1,3-dien aus dem Produktgemisch von Schritt (a).

12. Verfahren nach Anspruch 10 oder 11, ferner umfassend die Gewinnung von Trichlorethylen aus dem Produktgemisch von Schritt (a).

13. Verfahren nach einem der Ansprüche 10, 11 oder 12, ferner umfassend die Gewinnung von E- und Z-1,1,1,4,4,4-Hexafluor-2-chlor-2-buten aus dem Produktgemisch von Schritt (b).

14. Verfahren nach einem der Ansprüche 10, 11, 12 oder 13, ferner umfassend die Gewinnung von 1, 1, 1,4,4,4-Hexafluor-2-butin aus dem Produktgemisch von Schritt (c).

15. Verfahren nach einem der Ansprüche 10, 11, 12, 13 oder 14, ferner umfassend die Gewinnung von Z-1,1,1,4,4,4-Hexafluor-2-buten aus dem Produktgemisch von Schritt (d).

## Revendications

1. Procédé de production d'un mélange de produits comprenant le E- et le Z-1,1,1,4,4,4-hexafluoro-2-chloro-2-butène comprenant la mise en contact de 1,1,2,4,4-pentachlorobuta-1,3-diène avec HF en phase vapeur en présence d'une source de chlore et d'un catalyseur de fluoration et pour produire un mélange de produits comprenant le E- et le Z- 1, 1, 1,4,4,4-hexafluoro-2-chloro-2-butène.

2. Procédé selon la revendication 1, dans lequel le catalyseur de fluoration comprend un chlorure métallique ou un oxychlorure métallique, dans lequel le catalyseur est la source de chlore.

3. Procédé selon la revendication 1, dans lequel le catalyseur de fluoration comprend un halogénure métallique ou un oxyhalogénure métallique, dans lequel l'halogénure n'est pas un chlorure ou un oxyde métallique et la source de chlore est du chlore (Ch) de préférence
dans lequel le rapport du chlore sur le 1,1,2,4,4-pentachlorobuta-1,3-diène est de 1 à 10 (rapport molaire).

4. Procédé selon la revendication 1, dans lequel le procédé est effectué en présence de chlore (Ch), ou
dans lequel le rapport molaire de HF sur le 1,1,2,4,4-pentachlorobuta-1,3-diène est de 1 à 35, ou
dans lequel le procédé est effectué à une température dans l'intervalle de 250 à 425 °C, ou
dans lequel le procédé est effectué à une pression dans l'intervalle de 0 à 200 psi (0 à 1,4 MPa).

5. Procédé selon la revendication 1, comprenant en outre la production de 1,1,2,4,4-pentachlorobuta-1,3-diène par la mise en contact de trichloroéthylène avec un catalyseur de dimérisation comprenant du fer pour produire un mélange de produits comprenant le 1,1,2,4,4-pentachlorobuta-1,3-diène.

6. Procédé selon la revendication 5, dans lequel le trichloroéthylène est mis en contact avec un catalyseur de dimérisation comprenant du fer et du pentachloroéthane.

7. Procédé de production d'un mélange de produits comprenant le E- et le Z-1,1,1,4,4,4-hexafluoro-2-chloro-2-butène comprenant :
(a) la production de 1,1,2,4,4-pentachlorobuta-1,3-diène par la mise en contact de trichloroéthylène avec un catalyseur de dimérisation pour produire un mélange de produits comprenant le 1,1,2,4,4-pentachlorobuta-1,3-diène ; et
(b) la mise en contact du 1,1,2,4,4-pentachlorobuta-1,3-diène avec HF en phase vapeur en présence d'un catalyseur de fluoration comprenant un halogénure métallique pour produire un mélange de produits comprenant le E- et le Z-1,1,1,4,4,4-hexafluoro-2-chloro-2-butène.

8. Procédé de production d'un mélange de produits comprenant le E- et le Z-1,1,1,4,4,4-hexafluoro-2-chloro-2-butène comprenant :
(a) la production de 1,1,2,4,4-pentachlorobuta-1,3-diène par mise en contact de trichloroéthylène avec un catalyseur de dimérisation et du pentachloroéthane pour produire un mélange de produits comprenant le 1,1,2,4,4-pentachlorobuta-1,3-diène; et
(b) la mise en contact du 1,1,2,4,4-pentachlorobuta-1,3-diène avec HF en phase vapeur en présence d'un catalyseur de fluoration comprenant un halogénure métallique pour produire un mélange de produits comprenant le E- et le Z-1,1,1,4,4,4-hexafluoro-2-chloro-2-butène.

9. Procédé selon la revendication 7 ou 8, comprenant en outre la récupération du 1,1,2,4,4-pentachlorobuta-1,3-diène à partir du mélange de produits de l'étape (a), ou
comprenant en outre la récupération du trichloroéthylène à partir du mélange de produits de l'étape (a).

10. Procédé pour produire du Z-1,1,1,4,4,4-hexafluorobut-2-ène, comprenant :
(a) la mise en contact de trichloroéthylène avec un catalyseur de dimérisation pour produire un mélange de produits comprenant du 1,1,2,4,4-pentachlorobuta-1,3-diène ;
(b) la mise en contact du 1,1,2,4,4-pentachlorobuta-1,3-diène avec HF en phase vapeur en présence d'un catalyseur de fluoration comprenant un halogénure métallique pour produire un mélange de produits comprenant le E- et le Z-1,1,1,4,4,4-hexafluoro-2-chloro-2-butène ;
(c) la mise en contact du E- et du Z-1,1,1,4,4,4-hexafluoro-2-chloro-2-butène avec une base pour produire un mélange de produits comprenant du 1,1,1,4,4,4-hexafluoro-2-butyne; et
(d) la mise en contact du 1,1,1,4,4,4-hexafluoro-2-butyne avec H₂ pour produire un mélange de produits comprenant le Z-1,1,1,4,4,4-hexafluorobut-2-ène.

11. Procédé selon la revendication 10, comprenant en outre la récupération du 1,1,2,4,4-pentachlorobuta-1,3-diène à partir du mélange de produits de l'étape (a).

12. Procédé selon la revendication 10 ou 11, comprenant en outre la récupération du trichloroéthylène à partir du mélange de produits de l'étape (a).

13. Procédé selon l'une quelconque des revendications 10, 11 ou 12, comprenant en outre la récupération du E- et du Z-1,1,1,4,4,4-hexafluoro-2-chloro-2-butène à partir du mélange de produits de l'étape (b).

14. Procédé selon l'une quelconque des revendications 10, 11, 12 ou 13, comprenant en outre la récupération du 1,1,1,4,4,4-hexafluoro-2-butyne à partir du mélange de produits de l'étape (c).

15. Procédé selon l'une quelconque des revendications 10, 11, 12, 13 ou 14, comprenant en outre la récupération du Z-1,1,1,4,4,4-hexafluoro-2-butène à partir du mélange de produits de l'étape (d).
